Europäisches Patentamt

European Patent Office

Office européen des brevets

(11) Publication number: **0 293 077**

**A1**

# (12) EUROPEAN PATENT APPLICATION

(21) Application number: 88303467.0

(22) Date of filing: 18.04.88

(51) Int. Cl.⁴: **G01N 33/576** , **G01N 33/569**

(30) Priority: 24.04.87 JP 102949/87

(43) Date of publication of application:
30.11.88 Bulletin 88/48

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Fujisawa, Yukio
31-104, 1 Mikagenakamachi 4-chome
Higashinada-ku
Kobe Hyogo 658(JP)
Inventor: Onda, Haruo
201, 17-1 Namiki 3-chome Tsukuba
Ibaraki 305(JP)
Inventor: Hinuma, Shuji
D73-207, 18 Tsukumodai, 5-chome
Suita Osaka 565(JP)

(74) Representative: Lewin, John Harvey et al
Elkington and Fife High Holborn House 52/54
High Holborn
London WC1V 6SH(GB)

(54) **Assay for anti-pre-5 antibody.**

(57) An assay for anti-pre-S antibody in a serum specimen which comprises adding a carrier having Protein A bound thereto to a mixture containing the serum specimen and a pre-S peptide labeled with $^{125}$I, then separating the carrier from the mixture, and measuring radioactivity of the separated carrier is highly specific, highly sensitive and simply operable.

EP 0 293 077 A1

## Assay for Anti-pre-S Antibody

### Field of the Invention

The present invention relates to an assay for anti-pre-S antibody in a serum, more particularly to a method for determining an antibody to the pre-S region present at the N-terminal of the L-protein or the M-protein in hepatitis B virus (HBV) env proteins by a radioimmunoassay.

### Description of the Prior Art

It has been known that the pre-S antigen-anti-pre-S antibody system is induced in the sera of patients with acute hepatitis infected with HBV and developing the symptoms thereof, prior to the induction of the HBs antigen - anti-HBs antibody system, the HBc antigen - anti-HBc antibody system and the HBe antigen - anti-HBe antibody system [A. Robert Neurath, et al., Nature, 315, 154 (1985)]. It has been noted what kind of role is played by pre-S antigen and anti-pre-S antibody in respect to the completion of HBV infection, the mechanism by which the symptoms of type B hepatitis are developed, and the prevention thereof and the like. Particularly, it has recently been proven by experiments conducted by use of chimpanzees that antisera to a pre-S2 peptide in the pre-S region neutralize HBV [A. Robert Neurath et al., Vaccine, 4, 35 (1986)] and the development of the symptoms of acute hepatitis caused by HBV is prevented by a pre-S2 peptide vaccine [Y. Itoh et al., Proc. Natl. Acad. Sci. U.S.A., 83, 9174 (1986)]. It has further been reported that antibodies to a pre-S1 peptide in the pre-S region prevent the binding of HBV to cells derived from hepatoma [A. Robert Neurath et al., Cell, 46, 429 (1986)]. As the importance of anti-S antibody is thus recognized, there has been desired to develop a system by which anti-pre-S antibody in sera of hepatitis B patients or persons inoculated with the hepatitis B vaccine can be determined specifically and sensitively.

When the antibodies in human sera are determined by the usual enzyme immunoassay (EIA), a remarkably unspecific reaction is offten observed, greatly different from the case where sera of experimental animals are used. Therefore, the radioimmunoassay (RIA) is widely used for determination of antibody in human sera. The determination of human anti-pre-S antibody is also dependent on the RIA. For example, anti-pre-S antibody in sera of patients with acute type B hepatitis has been determined by detecting the antibody bound to a pre-S peptide in a solid phase by the antiglobulin method [A. Robert Neurath et al., Nature, 315, 154 (1985)]. The anti-pre-S antibody in human sera has been determined by the inhibition method by allowing human sera to react with an HBs antigen solution containing a definite amount of pre-S antigen, then binding the unreacted pre-S antigen-containing HBs antigen to beads coated with anti-pre-S antibody, and detecting it by $^{125}$I-labeled anti-HBs antibody [A. Budkowska et al., Hepatology, 6, 360 (1986)]. Further, with respect to the human antibody to the polymerized albumin receptor (PAR) in the pre-S2 region, the anti-PAR antibody has been determined by the inhibition method by allowing sera to react with PAR-containing HBs particles in a solid phase, then binding polymerized human serum albumin (poly-HsA) to the unreacted PAR, and detecting it by $^{125}$I-labeled monoclonal anti-poly-HSA antibody [H. Okamoto et al., Hepatology, 6, 354 (1986)]. However, in the antiglobulin method in which the antigen bound to the solid phase is allowed to react with a serum specimen and the antibody trapped by the solid phase is detected by RIA, unspecific reactions are observed. Accordingly, a diluted human serum is required to be used. Therefore, this method is unsuitable for detecting a low level of antibody titer. In the inhibition method in which the antigen is previously allowed to react with a serum specimen in a liquid phase and then the amount of unreacted antigen is determined by RIA, the antibody in the serum can be calculated since unspecific reactions are negligible, but the operations are complicated. Thus, the usual RIAS for determining human anti-pre-S antibody titer have merits and demerits. Therefore, the development of highly specific, highly sensitive and simply operable RIA has been awaited.

### Summary of the Invention

With this background, the present inventors undertook to develop a system by which anti-pre-S antibody in human sera can be determined highly specifically, highly sensitively and simply. As a result of their efforts, the present inventors have found a system which is not accompanied by any unspecific reaction and by which anti-pre-S antibody in a serum specimen can be sensitively determined, and thus completed the present invention.

According to the present invention, an assay for anti-pre-S antibody in a serum specimen is provided in which a carrier having Protein A bound thereto is added to a mixture containing the serum specimen and a pre-S peptide labeled with $^{125}$I, then the carrier is separated from the mixture, and the radioactivity of the separated carrier is measured.

As the above pre-S peptide, peptides containing a conserved region in the pre-S region among HBV subtypes such as adr, adw, ayw and ayr are suitable. There are preferably used a peptide comprising the following amino acid sequence from 21st to 47th in the pre-S1 region :

PLGFX$^1$PDHQLDPAFX$^2$ANX$^3$X$^4$NPDWDFNP

wherein X$^1$ represents F or L, X$^2$ represents G or R, X$^3$ represents S or T and X$^4$ represents N, T or A; a peptide comprising the following amino acid sequence from 25th to 42nd in the pre-S1 region:

X$^1$PDHQLDPAFX$^2$ANX$^3$X$^4$NPD

wherein X$^1$, X$^2$, X$^3$ and X$^4$ have the same meanings as defined above; a peptide comprising the following amino acid sequence from 8th to 34th in the pre-S2 region :

X$^5$HQX$^6$LX$^7$DPRVRGLYX$^8$PAGGSSSGTVNP

wherein X$^5$ represents F, L or P, X$^6$ represents A or T, X$^7$ represents L or Q and X$^8$ represents F or L; and a conjugate of one or more of these peptides with a carrier protein such as bovine serum albumin, thyroglobulin and keyhole limpet hemocyanin. A polypeptide containing the above pre-S1 and/or pre-S2 may also be used. The pre-S1 peptide, the pre-S2 peptide and the polypeptide containing pre-S1 and pre-S2 can be synthesized by chemical syntheses or gene engineering procedures.

The above pre-S peptide can be labeled with $^{125}$I by the method in which ICl is used [S. Zappacosta and G. Rossi, Immunochemistry 4, 122 (1967)], the method in which chloramine-T is used [N. R. Klinman and R. B. Taylor, Clin. Exp. Immunol. 4, 473 (1969)], the method in which Bolton-Hunter reagent is used [A. E. Bolton and W. M. Hunter, Biochem. J. 133, 529 (1973)] and the like.

As the carrier to which Protein A is to be bound, agarose gel (Sepharose) is usually used, because of its easiness of separation from the liquid phase and washing. As the carrier to which Protein A has been bound, commercially available Protein A-Sepharose CL-4B (Pharmacia Inc.) and the like are preferably used.

The procedures of the assay for anti-pre-S antibody according to the present invention will hereinafter be described.

Step 1

A serum specimen (or a diluted solution thereof) and a $^{125}$I-labeled pre-S peptide are mixed with each other.

If anti-pre-S antibody is present in the serum specimen, a complex of anti-pre-S antibody and pre-S peptide is formed by the antigen-antibody reaction.

The serum used is prepared by a known method from blood collected. Also, an immunoglobulin G fraction prepared from the serum may be used. For dilution of the mixture, about 10 to 100 mM phosphate buffer (pH 7.0 to 8.0), sodium chloride solution or water is usually used. The mixture is usually allowed to stand or stirred, at 4 to 45°C for 1 to 20 hours. In order to inhibit protease activity in the serum, an enzyme inhibitor may be added.

Step 2

A carrier to which Protein A has been bound is added to the mixture obtained in Step 1.

Protein A has an affinity for the Fc region of immunoglobulin G. Therefore, if anti-pre-S antibody is present in the serum specimen, a complex of carrier - Protein A - anti-pre-S antibody - $^{125}$I-labeled pre-S peptide is formed. After the addition of the carrier, the mixture is usually stirred at room temperature for about 0.5 to 3 hours.

Step 3

The carrier is separated from the mixture.

Usually, the carrier is separated from the mixture by centrifugation or filtration. The separated carrier is washed, and the unreacted $^{125}$I-labeled pre-S peptide contained therein is removed.

Step 4

The radioactivity of the carrier is determined.

For this determination, any customary radiation detector can be used. Anti-pre-S antibody in the serum specimen can be determined quantitatively by comparison with anti-pre-S antibody-containing sera of various known concentrations, as the standard sera, measured in a similar manner as above.

When anti-pre-S antibody is not present in the serum specimen, only immunoglobulin G which does not react with the pre-S peptide is bound to Protein A on the separated carrier. When anti-pre-S antibody is present in the serum specimen, anti-pre-S antibody and immunoglobulin G react competitively with Protein A. Further, anti-pre-S antibody and the $^{125}$I-labeled pre-S peptide react with each other, and $^{125}$I is trapped on the carrier. Thus, the carrier becomes radioactive.

The assay for anti-pre S antibody according to the present invention is highly specific, highly sensitive and simply operable.

When amino acids are indicated by the abbreviations given in a single letter in this specification, the conventional abbreviations in the art are used and some examples are as follows. The amino acids represent the L-forms, unless otherwise specified.

A: Alanine
C: Cysteine
D: Aspartic acid
E: Glutamic acid
F: Phenylalanine
G: Glycine
H: Histidine
I: Isoleucine
K: Lysine
L: Leucine
M: Methionine
N: Asparagine
P: Proline
Q: Glutamine
R: Arginine
S: Serine
T: Threonine
V: Valine
W: Tryptophan
Y: Tyrosine

Description of the preferred Embodiment

The present invention will hereinafter be described in detail with the following Reference Examples and Examples. It is understood that these examples are not intended to limit the scope of the inventions.

Reference Example 1

Synthesis of Pre-S2 Peptide,
FHQALLDPRVRGLYFPAGGSSSGTVNP

The pre-S2 peptide described above was synthesized by the solid phase method by using a Model 430A Peptide Synthesizer (Applied Biosystems Inc., U.S.A.).

Starting from 600 mg of a Boc. Pro. Pam resin (Pam: 4-oxymethylphenylacetamide), the reaction was conducted according to the program to obtain 2.5 g of a peptide resin. Then, 612 mg of the resin thus obtained was treated with 6 ml of anhydrous hydrogen fluoride in the presence of 0.6 ml of anisole at $0\degree$ C for 1 hour. The resulting free peptide was purified by using a PD-10 column (Pharmacia Inc., Sweden), Amberite IRA-400 (acetyic acid type)(Rohm & Haas Inc., U.S.A.), and then a reversed phase high performance liquid chromatography A347-7 (S-70DS) YMC (Yamamura Chemical Co.). The yield was 50 mg.

Analytical amino acid values of the acid-hydrolysate (6N HCl, $110\degree$ C, 24 hours) were as follows:

Asp, 1.9 (2); Thr, 0.9 (1); Ser, 2.7 (3); Glu, 1.1 (1); Gly, 4.3 (4); Ala, 2.2 (2); Val, 2.1 (2): Leu, 3.0 (3); Tyr, 1.1 (1); Phe, 2.0 (2); His, 1.0 (1); Arg, 2.0 (2); and Pro, 2.8 (3). Recovery 78.9%. The theoretical values were designated in parentheses.

$[\alpha]_D^{25}$ - 69.8 (c = 0.1, in 5% acetic acid)

Reference Example 2

Synthesis of Pre-S1 Peptide,
PLGFFPDHQLDPAFGANSNNPDWDFNP

The pre-S1 peptide described above was prepared in a similar manner as described in Reference Example 1.

Example 1

Assay for Anti-Pre-S2 Antibody

1) $^{125}$I-Labelling of Pre-S2 Peptide

1 mCi of Bolton-Hunter reagent (New England Nuclear Inc.) was allowed to react with the pre-S2 peptide described in Reference Example 1 [20 $\mu$g/20$\mu$l of phosphate-buffered saline (PBS: 29.2 g of NaCl, 0.2 g of KCl, 0.2 g of $KH_2PO_4$ and 1.45 g of $Na_2HPO_4$ were brought to 1 l with distilled water)] at $4\degree$ C for 3 hours, followed by addition of 180 $\mu$l of 2M glycine, and the reaction was further continued at $4\degree$ C for 15 minutes. After the completion of the reaction, the reaction mixture was subjected to gel filtration by using a PD 10 column (Pharmacia Inc.), followed by elution of $^{125}$I-pre-S peptide with 2% bovine serum albumin (BSA)-PBS-0.05% $NaN_3$ to remove unreacted Bolton-Hunter reagent. The $^{125}$I-labeled pre-S2 peptide fraction was further diluted 4 times with 2% BSA-PBS-0.05% $NaN_3$, and the dilution was used for determination of anti-pre-S2 antibody.

2) Assay for Anti-Pre-S2 Antibody

50 $\mu$l of a human serum specimen was allowed to react with 10$\mu$l of the $^{125}$I-labeled pre-S2 peptide obtained above at $4\degree$ C overnight, and then 200$\mu$l of a Protein A-Sepharose suspension which was prepared by suspending Protein A-Sepharose in 3% gelatin - 0.1 M phosphate buffer (pH 8.0) to give a concentration of 50% (V/V) was added thereto, followed by stirring at room temperature for 1 hour. After the reaction, centrifugation (10,000 × g, for 15 seconds) was carried out to collect a precipitate. Then, 1.0 ml of a 0.1 M

5

phosphate buffer (pH 8.0) was added thereto and the mixture was stirred. Thereafter, centrifugation (10,000 × g, for 15 seconds) was again carried out to collect a precipitate. The radioactivity of the precipitate fraction was determined by an auto-gamma counter. Anti-pre-S2 antibody in HB Globulin - Nichiyaku (Nippon Seiyaku Co.), an immunoglobulin preparation, showed about $1.8 \times 10^4$ by cpm, when determined by this method. Anti-pre-S2 antibody in human sera was determined by this method. The resutls are shown in Table 1.

Example 2

1) $^{125}$I-Labelling of Pre-S1 Peptide

Pre-S1 peptide described in Reference Example 2 was labeled with $^{125}$I in the same manner as described in 1) of Example 1.

2) Assay for Anti-Pre-S1 Antibody

Anti-pre-S1 antibody was determined in the same manner as described in 2) of Example 1, with the exception that $^{125}$I-labeled pre-S1 peptide was used. Anti-pre-S1 antibody in HB Globulin - Nichiyaku (Nippon Seiyaku Co.) showed about $1.3 \times 10^4$ by cpm, when determined by this method. Anti-pre-S1 antibody in human sera was determined by this method. The results are shown in Table 2.

Table 1   Detection of anti-pre-S2 antibody in Human sera

| Specimen | | Radioactivity bound to Protein A-Sepharose (cpm) * |
|---|---|---|
| Healthy human sera | A | 1,602 |
| negative for anti- | B | 1,979 |
| HBs antibody | C | 2,015 |
| Human sera posi- | D | 5,393 |
| tive for anti-HBs | E | 10,189 |
| antibody | F | 12,572 |

* The radioactivity of $^{125}$I-labeled pre-S2 peptide (10μl) used for determination of anti-pre-S2 antibody was 372,222 cpm.

Table 2   Detection of anti-pre-S1 antibody in human sera

| Specimen | | Radioactivity bound to Protein A-Sepharose(cpm)* |
|---|---|---|
| Healthy human sera | G | 1,825 |
| negative for anti- | H | 2,074 |
| HBs antibody | I | 2,163 |
| Human sera posi- | J | 8,505 |
| tive for anti-HBs | K | 13,460 |
| antibody | L | 12,629 |

* The radioactivity of $^{125}$I-labeled pre-S1 peptide (10μl) used for determination of anti- pre-S1 antibody was 395,605 cpm.

## Claims

(1) An assay for anti-pre-S antibody in a serum specimen which comprises adding a carrier having Protein A bound thereto to a mixture containing the serum specimen and a pre-S peptide labeled with $^{125}$I, then separating the carrier from the mixture, and measuring radioactivity of the separated carrier.

(2) The assay as claimed in claim 1, wherein the pre-S peptide has the following amino acid sequence: FHQALLDPRVRGLYFPAGGSSSGTVNP, or
PLGFFPDHQLDPAFGANSNNPDWDFNP.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | EP-A-0 154 902 (NEW YORK BLOOD CENTER, INC.)<br>* claims 136-139; pages 91-93, example 24, claims 1-8,21-23 * | 1 | G 01 N 33/576<br>G 01 N 33/569 |
| A | | 2 | |
| Y | EP-A-0 080 109 (NEW YORK BLOOD CENTER, INC.)<br>* page 19, example 4 * | 1 | |
| A | GB-A-2 026 691 (ABBOTT LABORATORIES)<br>* claims 1-15 * | 1,2 | |

**TECHNICAL FIELDS SEARCHED (Int. Cl.4)**

G 01 N 33/00
C 12 P 21/00
A 61 K 39/00
C 12 N 15/00
C 07 K 7/00

The present search report has been drawn up for all claims

| Place of search | Date of completion of the search | Examiner |
|---|---|---|
| BERLIN | 26-07-1988 | DE KOK A.J. |